# EUROPEAN PATENT APPLICATION

(11) **EP 3 785 611 A1**
(43) Date of publication of application: **03.03.2021**
(21) Application number: 20192745.6
(22) Date of filing: 25.08.2020
(51) Int. Cl.: A61B 5/00, G16H 50/20, G16H 50/30

(54) **SEPSIS MONITORING SYSTEM**

(30) Priority: 30.08.2019 US 201962893985 P
(71) Applicant: Hill-Rom Services, Inc., Batesville, IN 47006-9167 (US)
(72) Inventor: CHUNG, Chiew Yuan, Batesville, IN 47006-9167 (US); DE BIE, Johannes, Batesville, IN 47006-9167 (US); FITZGIBBONS, Stacey A., Batesville, IN 47006-9167 (US); KAYSER, Susan, Batesville, IN 47006-9167 (US); MEYERSON, Craig M., Batesville, IN 47006-9167 (US); NOFFKE, Patrick James, Batesville, IN 47006-9167 (US); SEPEHR, Reyhaneh, Batesville, IN 47006-9167 (US); SHI, Yuan, Batesville, IN 47006-9167 (US); URRUTIA, Eugene, Batesville, IN 47006-9167 (US)
(74) Representative: Findlay, Alice Rosemary

(57) **Abstract**

A sepsis monitoring system determines an initial sepsis risk assessment score, and automatically and continuously updates the sepsis risk assessment score using a sepsis risk assessment model that receives vital signs data, electronic medical records data, and admissions data to continuously update the sepsis risk assessment score. The system determines whether the patient is likely to develop sepsis based on the updated sepsis risk assessment score, and in response to determining that the patient is likely to develop sepsis, generates a notification to drive an early intervention by one or more caregivers.

## Description

Sepsis is a life-threatening condition that occurs when the body's response to infection causes injury to its own tissues and organs. Sepsis develops when a pathogen is released into the bloodstream and causes inflammation throughout the entire body.

Sepsis at its earliest stages is usually reversible with antibiotics, fluids, and other supportive medical interventions. However, as time progresses the risk of dying increases substantially. Therefore, early detection of sepsis is desirable.

In general terms, the present disclosure relates to a system that continuously updates a sepsis risk assessment score to drive an early intervention by one or more caregivers.

In one aspect, a sepsis monitoring system comprises a processor and a non-transitory computer-readable storage medium storing instructions that, when executed by the processor, cause the sepsis monitoring system to: determine an initial sepsis risk assessment score; automatically and continuously update the sepsis risk assessment score using a sepsis risk assessment model that receives vital signs data, electronic medical records data, and admissions data to continuously update the sepsis risk assessment score; determine whether the patient is likely to develop sepsis based on the updated sepsis risk assessment score; and in response to determining that the patient is likely to develop sepsis, generate a notification to drive an early intervention by one or more caregivers.

In another aspect, a method of providing an early intervention for mitigating risks associated with sepsis comprises determining an initial sepsis risk assessment score; continuously receiving measured vital signs from a vital signs monitoring device; automatically and continuously determining an updated sepsis risk assessment score based on at least the measured vital signs; determining whether a patient is likely to develop sepsis based on the updated sepsis risk assessment score; and in response to determining that the patient is likely to develop sepsis, generating a notification to drive an early intervention by one or more caregivers.

The invention will now be further described by way of example with reference to the accompanying drawings, in which:
FIG. 1 is a block diagram schematically illustrating a healthcare facility that includes a sepsis monitoring system.
FIG. 2 is a block diagram schematically illustrating healthcare information systems.
FIG. 3 is a schematic block diagram of the sepsis monitoring system.
FIG. 4 illustrates an example method of providing an early intervention.
FIG. 5 illustrates an example method of building a sepsis risk assessment model.
FIG. 6 is a schematic diagram of a model building engine.
FIG. 7 is a schematic diagram of a data evaluation engine.
FIG. 8 illustrates example physical components of a computing device.

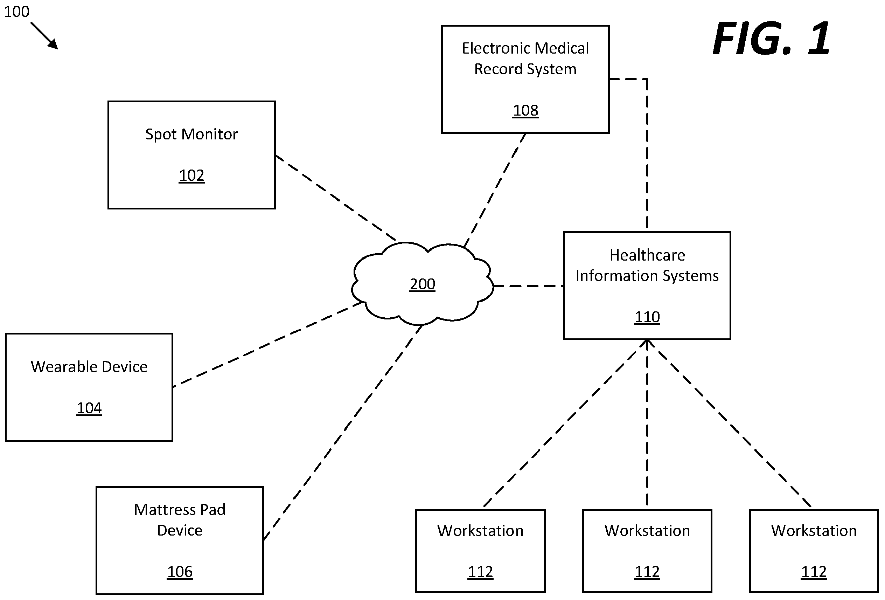

FIG. 1 is a block diagram schematically illustrating a healthcare facility 100 that includes a sepsis monitoring system 200. The sepsis monitoring system 200 is a clinical decision support tool that automatically and continuously updates a sepsis risk assessment for a patient admitted in the healthcare facility 100. As used herein, the term "continuous" or "continuously" includes near real-time such that the sepsis risk assessment is updated without perceivable lag.

The sepsis monitoring system 200 is operably connected to vital signs monitoring devices including at least one of a spot monitor device 102, a wearable device 104, and a mattress pad device 106. The sepsis monitoring system 200 is also operably connected to an electronic medical record system 108 and healthcare information systems 110. The healthcare information systems 110 is connected to a plurality of workstations 112.

The sepsis monitoring system 200 retrieves data from the spot monitor device 102, wearable device 104, mattress pad device 106, electronic medical record system 108, and healthcare information systems 110 to continuously determine the sepsis risk assessment.

In some embodiments, the sepsis risk assessment is transmitted from the sepsis monitoring system 200 to the healthcare information systems 110. Thereafter, the healthcare information systems 110 transmit the sepsis risk assessment for display on the workstations 112. In alternative embodiments, the sepsis risk assessment is transmitted directly from the sepsis monitoring system 200 to the workstations 112 without using the healthcare information systems 110 as an intermediary for transmitting the sepsis risk assessment to the workstations 112.

In alternative embodiments, the healthcare information systems 110 receive data from the sepsis monitoring system 200 (including vital signs data from at least one of the spot monitor device 102, wearable device 104, and mattress pad device 106 and data from the electronic medical record system 108), and the healthcare information systems 110 calculate the sepsis risk assessment, and transmit the sepsis risk assessment for display on the workstations 112. In these embodiments, the sepsis monitoring system 200 acts as a gateway for collecting and transmitting the continuous vital signs to the healthcare information systems 110.

The sepsis monitoring system 200 uses a sepsis risk assessment model that receives as an input vital signs data acquired from at least one of the spot monitor device 102, wearable device 104, mattress pad device 106, as well as data from the electronic medical record system 108 and/or healthcare information systems 110 to generate the sepsis risk assessment as an output. The vital signs data acquired from at least one of the spot monitor device 102, wearable device 104, mattress pad device 106 includes, without limitation, heart rate, respiratory rate, blood pressure, blood oxygen saturation (e.g., SpO2), electrocardiogram data (EKG or ECG), electroencephalography data (EEG), and objective level of pain.

In one embodiment, the sepsis monitoring system 200 is a cloud based system that is hosted over the Internet. In this embodiment, notifications from the sepsis monitoring system 200 are sent to the healthcare information systems 110 and workstations 112 via the Internet.

In an alternative embodiment, the sepsis monitoring system 200 is part of a local area network. In this example embodiment, notifications from the sepsis monitoring system 200 are sent to the healthcare information systems 110 and workstations 112 via the local area network.

The vital signs monitoring devices, including the spot monitor device 102, wearable device 104, and mattress pad device 106, are each configured to continuously, without interruption, measure the vital signs of a patient in the healthcare facility 100 before, during, and after patient rounds by a caregiver. The continuous vital sign monitoring can help provide early identification of patient deterioration and drive early intervention by the caregiver.

The spot monitor device 102 can be mounted onto a mobile stand or can be wall mounted. The spot monitor device 102 measures heart rate, respiratory rate, blood pressure, temperature, blood oxygen saturation, and the like. In some examples, the spot monitor device 102 includes WiFi®, Ethernet, or Bluetooth® connectivity to EMR systems.

The wearable device 104 is a wearable biosensor that continuously measures the vital signs of the patient. In some examples, the wearable device 104 is a specialized vital signs patch (VSP) that continuously measures vital signs including heart rate, respiratory rate, blood pressure, temperature, blood oxygen saturation, and the like.

The mattress pad device 106 is designed for placement under the mattress of a bed where a patient is resting to continuously monitor heart rate and respiratory rate of the patient, and additional physiological parameters such as patient weight and posture.

The electronic medical record system 108 stores electronic medical records (EMRs). Each EMR contains the medical and treatment history of a patient in the healthcare facility 100. In some embodiments, the electronic medical record system 108 receives vital signs data and other physiological parameter data measured by the spot monitor device 102, wearable device 104, and mattress pad device 106 via the sepsis monitoring system 200.

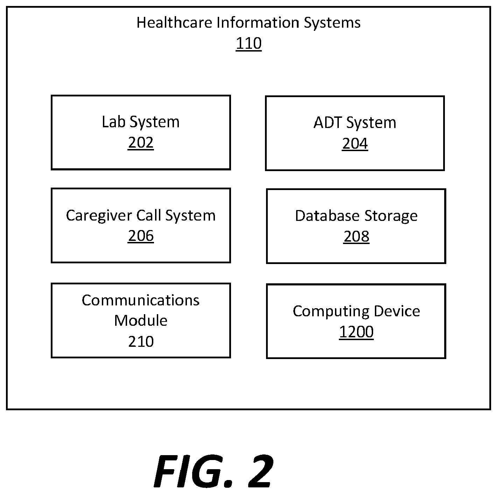

The healthcare information systems 110, which are described in more detail with reference to FIG. 2, include various systems that are operational within the healthcare facility 100. The healthcare information systems 110 are communicatively connected to the workstations 112. In the example embodiment of FIG. 1, the electronic medical record system 108 is depicted as separate from the healthcare information systems 110. In alternative example embodiments, the electronic medical record system 108 is part of the healthcare information systems 110.

The workstations 112 include computing devices such as smartphones, tablet computers, laptops, desktop computers, and the like. The workstations 112 are used by caregivers in the healthcare facility 100 to access the healthcare information systems 110, receive notifications from the sepsis monitoring system 200, and access electronic medical records from the electronic medical record system 108. The number of workstations 112 present in the healthcare facility 100 may vary according to the needs of the healthcare facility 100.

The sepsis monitoring system 200 communicates with the spot monitor device 102, wearable device 104, mattress pad device 106, electronic medical record system 108, and healthcare information systems 110, and workstations 112 through a wireless connection, a wired connection, or a combination of wireless and wired connections. Examples of wireless connections include Wi-Fi communication devices that utilize wireless routers or wireless access points, cellular communication devices that utilize one or more cellular base stations, Bluetooth, ANT, ZigBee, medical body area networks, personal communications service (PCS), wireless medical telemetry service (WMTS), and other wireless communication devices and services.

FIG. 2 is a block diagram schematically illustrating the healthcare information systems 110. As shown in FIG. 2, the healthcare information systems 110 include a lab system 202, an Admission, Discharge, and Transfer (ADT) system 204, a caregiver call system 206, a database storage 208, a communications module 210, and a computing device 1200.

The lab system 202 monitors lab results by recording the date and time a patient sample is taken, sending updates to the caregiver regarding the lab results including when the lab results are ready, and displaying the lab results for review by the caregiver.

The ADT system 204 provides real-time information on each patient admitted to the healthcare facility 100 including the patient's name, address, gender, room assignment within the healthcare facility 100, date and time when admitted to and discharged from the healthcare facility 100, and whether the patient has been transferred to another room or department.

The caregiver call system 206 generates alerts that are triggered by one or more rules. The alerts are sent to the workstations 112 to notify the caregivers for need to perform critical tasks. The alerts can be generated based on data from the vital signs monitoring devices including the spot monitor device 102, wearable device 104, and mattress pad device 106, the electronic medical record system 108, lab system 202, ADT system 204, etc. As an illustrative example, patient early warning scores (EWS) when above a predetermined threshold trigger an alert from caregiver call system 206 that is sent to a workstation 112 associated with a caregiver so that the caregiver is notified of the need to perform critical tasks based on the elevated EWS.

In some embodiments, the caregiver call system 206 generates notifications based on the sepsis risk assessment from the sepsis monitoring system 200 which are sent to the workstations 112. In alternative embodiments, the sepsis risk assessment and associated notifications are transmitted directly from the sepsis monitoring system 200 to the workstations 112 without using the caregiver call system 206 as an intermediary. In yet further alternative embodiments, the caregiver call system 206 determines the sepsis risk assessment.

The database storage 208 stores data received from the sepsis monitoring system 200 and electronic medical record system 108. The database storage 208 can also store data from the vital signs monitoring devices including the spot monitor device 102, wearable device 104, mattress pad device 106. In some example embodiments, the database storage 208 stores algorithms and models that are used by the caregiver call system 206 to determine the sepsis risk assessment.

The communications module 210 enables the various components within the healthcare information systems 110 to communicate with each other, and also with the sepsis monitoring system 200, electronic medical record system 108, and workstations 112.

The communications module 210 enables the caregiver call system 206 to act as a dispatch system to instruct caregivers when an intervention is warranted. In some examples, the communications module 210 provides a communication link, such as audio or video, between the patient and caregivers. Communication devices including telephone and other voice communication devices worn by the caregivers enable the communications module 210 to provide direct communication between the caregivers and the patient.

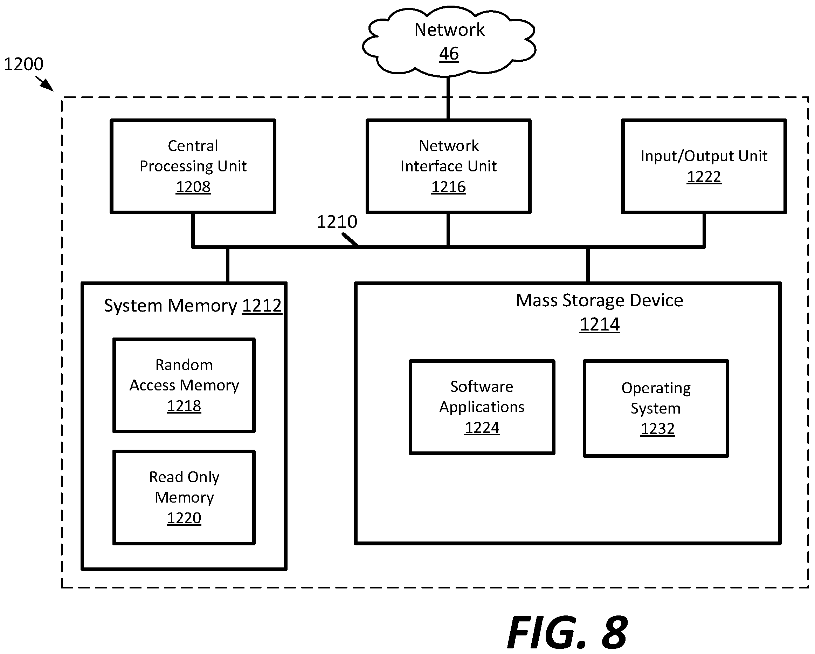

The computing device 1200 processes data from the database storage 208, and communicates with the lab system 202, ADT system 204, caregiver call system 206, and communications module 210. The computing device 1200 includes at least one processor that executes instructions to implement one or more of the methods described herein. The computing device 1200 is described in more detail with reference to FIG. 8.

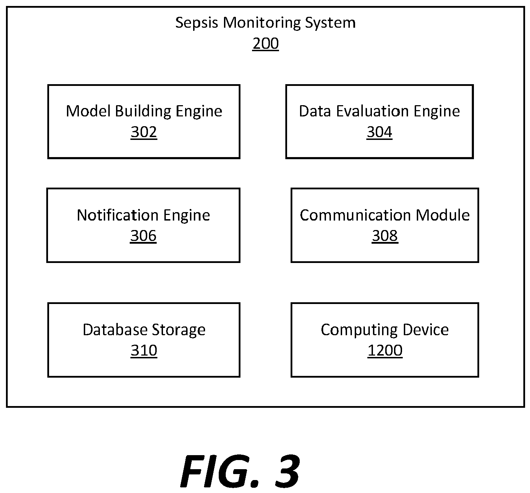

FIG. 3 is a schematic block diagram of the sepsis monitoring system 200. In a general sense, the sepsis monitoring system 200 is used to automatically and continuously estimate a likelihood for a given patient to develop sepsis. This likelihood can be used, as described herein, to mitigate risks and costs associated with sepsis through early intervention by caregivers. As an illustrative example, the sepsis monitoring system 200 can be used to predict a likelihood of the patient developing sepsis 6-12 hours before the onset of sepsis. The sepsis monitoring system 200 includes a model building engine 302, a data evaluation engine 304, a notification engine 306, a communications module 308, a database storage 310, and a computing device 1200.

The database storage 310 stores data from the electronic medical record system 108, healthcare information systems 110, and the vital signs monitoring devices including the spot monitor device 102, wearable device 104, and mattress pad device 106. In some examples, the database storage 310 is a critical data repository (CDR). In some embodiments, the data stored in the database storage 310 is used for machine learning and algorithm development.

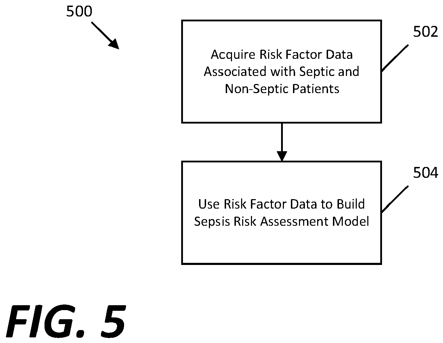

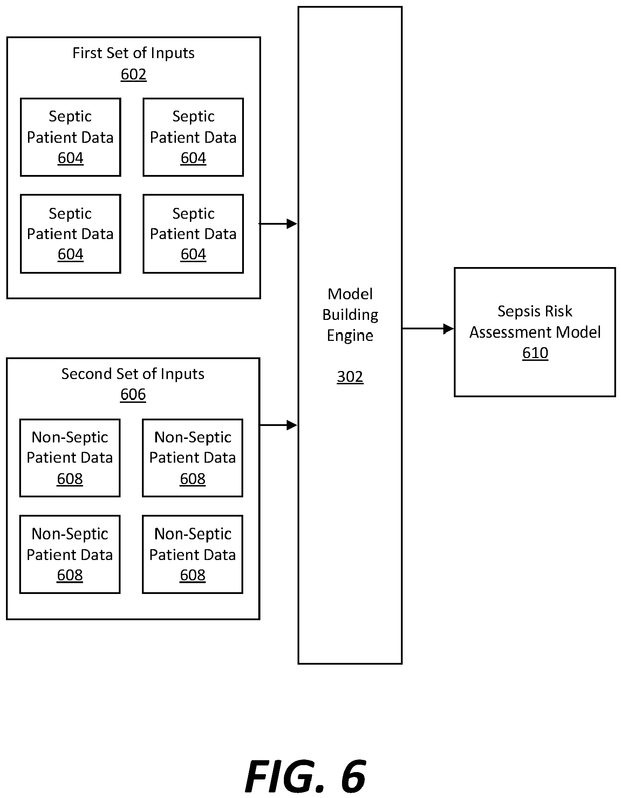

The model building engine 302 operates to build a model that can be used by the sepsis monitoring system 200 to automatically and continuously estimate a likelihood for a given patient to develop sepsis. The model building engine 302 uses the data stored in the database storage 310 to build the model. The model building engine 302 uses one or more machine learning techniques to build the model. Example methods performed by some embodiments of the model building engine 302 are illustrated and described with respect to FIGS. 5 and 6.

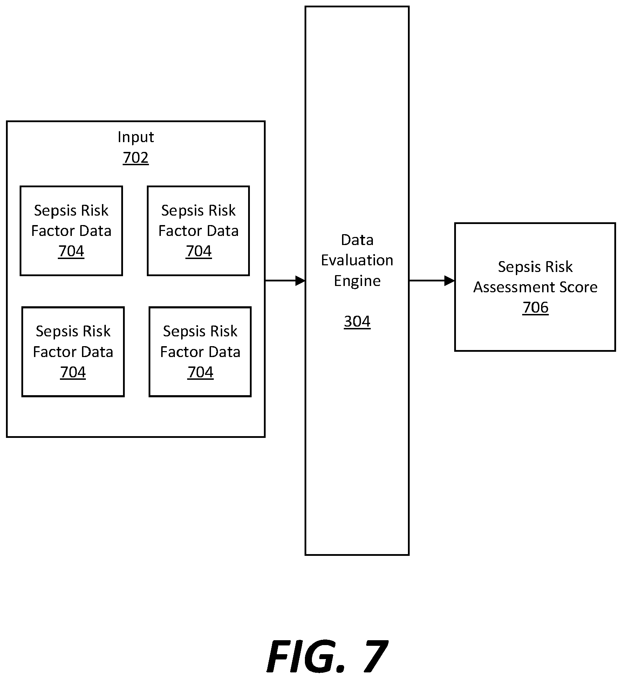

The data evaluation engine 304 operates to continuously evaluate data from at least one vital signs monitoring device such as, without limitation, at least one of the spot monitor device 102, wearable device 104, mattress pad device 106, and the like to determine a sepsis risk assessment score. The data evaluation engine 304 also operates to continuously evaluate data from the electronic medical record system 108 and healthcare information systems 110 to determine a sepsis risk assessment score. The data evaluation engine 304 uses the model built by the model building engine 302 to automatically and continuously determine the sepsis risk assessment score. The sepsis risk assessment score predicts whether a patient is likely to develop sepsis. Example methods performed by some embodiments of the data evaluation engine 304 are illustrated and described in more detail with respect to at least FIG. 7.

The notification engine 306 operate to generate one or more types of notifications to alert caregivers that the patient is likely to develop sepsis when the sepsis risk assessment score exceeds a threshold value, or if the sepsis risk assessment score is classified as a high risk or medium risk. The notification drives early intervention by the caregivers to mitigate the risks and costs associated with sepsis. The notification generated by the notification engine 306 may be delivered in any suitable form, including audible, visual, and textual such as a text message, pager message, email, or other form of alert, such as a message on the workstations 112.

The communications module 308 enables the sepsis monitoring system 200 to communicate with the vital signs monitoring devices including the spot monitor device 102, wearable device 104, and mattress pad device 106, and with the electronic medical record system 108, healthcare information systems 110, and workstations 112 in the healthcare facility 100. The sepsis monitoring system 200 can communicate with these devices and systems through a wireless connection, a wired connection, or a combination of wireless and wired connections.

The computing device 1200 processes the data from the database storage 310, and communicates with the model building engine 302, data evaluation engine 304, notification engine 306, and communications module 308. The computing device 1200 includes at least one processor that executes instructions to implement one or more of the methods described herein.

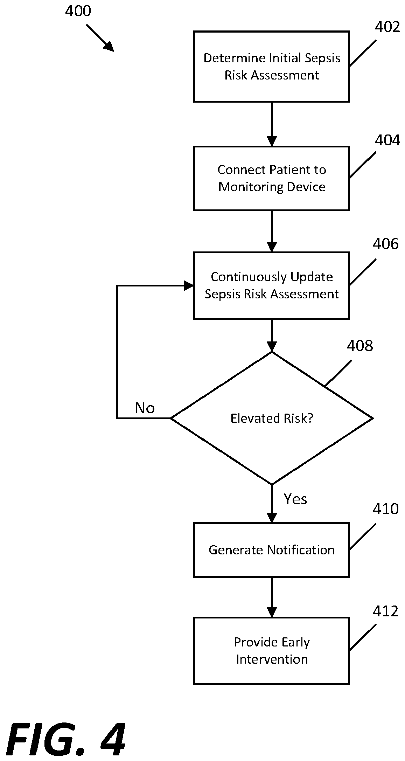

FIG. 4 illustrates an example method 400 of providing an early intervention for mitigating the risks and costs associated with sepsis. Referring now to FIG. 4, the method 400 at operation 402 includes determining an initial sepsis risk assessment score for a patient admitted to a healthcare facility. The sepsis risk assessment score predicts a likelihood of the patient developing sepsis while admitted in the facility. The score can be classified as low risk, medium risk, or high risk. Alternatively, the score can be numerical such as based on a scale between 0-100. A sepsis risk assessment model is used to determine the sepsis risk assessment score.

In some examples, the sepsis risk assessment score is displayed in a mobile platform operable on a portable device such as a tablet computer, smartphone, and the like. In addition to, or as an alternative to displaying the sepsis risk assessment score in the mobile platform, the sepsis risk assessment score can also be displayed as an EMR plug-in.

Next, the method 400 at operation 404 includes connecting the patient to at least one vital signs monitoring devices such as a spot monitor device, wearable device, or mattress pad device to continuously measure the vital signs of the patient.

Next, the method 400 at operation 406 includes automatically and continuously updating the sepsis risk assessment score for the patient. The sepsis risk assessment model is used to automatically and continuously update the sepsis risk assessment score.

In some embodiments, the sepsis risk assessment score is automatically and continuously updated using the continuously measured vital signs of the patient from at least one of the vital signs monitoring devices, as well as using additional physiological parameters and information from an electronic medical record system. When it is not possible to continuously measure the vital signs of the patient, the sepsis risk assessment score is automatically and continuously updated using data from the electronic medical record system.

At operation 408, the method 400 determines whether the patient is likely to develop sepsis based on the continuously updated sepsis risk assessment score. If the sepsis risk assessment score is less than a threshold value, or if the score is classified as a low risk (i.e., "No" in operation 408), the method 400 returns to continuously updating the sepsis risk score (i.e., operation 406). If the sepsis risk assessment score exceeds a threshold value, or if the score is classified as a high risk or medium risk (i.e., "Yes" in operation 408), a notification is generated at operation 410 to alert the caregivers that the patient is likely to develop sepsis.

At operation 410, a notification is delivered in any suitable form. For example, the notification can include audible, visual, and textual alerts such as text messages, pager messages, emails, or other forms of an alert, such as a message on a display device that is viewable by one or more caregivers. The notification can indicate that a certain action should be taken by a caregiver. For example, the notification can indicate that the caregiver should go check on the patient in-person, confirm the vital signs data, or order a lactate lab.

In some examples, the notification is displayed in a mobile platform operable on a portable device such as a tablet computer, smartphone, and the like. In addition to, or as an alternative to displaying the notification in the mobile platform, the notification is also displayed as an EMR plug-in that is accessible from a workstation.

At operation 412, the notification from operation 410 drives an early intervention to treat the patient and mitigate the risks and costs associated with sepsis. The early intervention can include providing antibiotics, fluids, taking additional laboratory tests, and other supportive medical interventions.

FIG. 5 illustrates a method 500 of building the sepsis risk assessment model for automatically and continuously determining a sepsis risk assessment score that is performed by some embodiments of the model building engine 302 (see FIG. 3). The method 500 can be used, for example, to build a model for determining an initial sepsis risk assessment score for a patient admitted to a healthcare facility such as in operation 402 of the method 400 (see FIG. 4) and also for automatically and continuously updating the sepsis risk assessment score for the patient such as in operation 406 of the method 400 described above (see FIG. 4).

At operation 502, sepsis risk factor data is acquired. The sepsis risk factor data includes vital signs data (e.g., data acquired from at least one of the vital signs monitoring devices of FIG. 1), electronic medical records data (e.g., data acquired from the EMR system 108 of FIG. 1), laboratory data (e.g., data acquired from the lab system 202 of FIG. 2), and admissions data (e.g., data acquired from the ADT system 204 of FIG. 2). The sepsis risk factor data is associated with septic patients and with non-septic patients. In some embodiments, the communications module 308 of the sepsis monitoring system 200 is utilized to acquire the sepsis risk factor data from the data sources within the healthcare facility 100.

At operation 504, the sepsis risk assessment model is built using the acquired sepsis risk factor data. In some embodiments, the sepsis risk assessment model is initially developed using a retrospective analysis of a database that includes the EMR data, lab data, ADT data, and vital signs data. In some embodiments, the database storage 310 of the sepsis monitoring system 200 is utilized to store the EMR data, lab data, ADT data, and vital signs data for performing the retrospective analysis. The model building engine 302 of the sepsis monitoring system 200 is utilized to build the sepsis risk assessment model.

FIG. 6 is a schematic diagram of the model building engine 302 that receives first and second sets of inputs 602, 606 to build a sepsis risk assessment model 610 as an output. The first set of inputs 602 include acquired sepsis risk factor data 604 associated with septic patients. The second set of inputs 606 include acquired sepsis risk factor data 608 associated with non-septic patients. In some embodiments, the acquired sepsis risk factor data 604, 608 is seed data used by the model building engine 302 to build the sepsis risk assessment model 610.

The acquired sepsis risk factor data 604 can be used as positive training examples while the acquired sepsis risk factor data 608 can be used as negative training examples to build the sepsis risk assessment model 610 using one or more machine learning techniques. In some embodiments, the sepsis risk assessment model 610 is built using supervised machine learning techniques such as a logistic regression. In alternative embodiments, the sepsis risk assessment model 610 is built using unsupervised machine learning techniques such as neural networks. Also, the sepsis risk assessment model 610 can be built by tree based methods such as gradient boosting machine learning techniques, and the like.

FIG. 7 is a schematic diagram of the data evaluation engine 304. The data evaluation engine 304 receives as an input 702 sepsis risk factor data 704 for a given patient. The data evaluation engine 304 uses the sepsis risk assessment model 610 to generate as an output a sepsis risk assessment score 706 that predicts whether the patient is likely to develop sepsis. The sepsis risk factor data 704 is automatically and continuously evaluated by the data evaluation engine 304 using the sepsis risk assessment model 610 to continuously determine the sepsis risk assessment score 706 while the patient is admitted to the healthcare facility. In certain embodiments, the sepsis risk assessment score 706 is calculated by the spot monitor 102 of FIG. 1. Alternatively, the sepsis risk assessment score 706 can be calculated by the sepsis monitoring system 200 which is a cloud based system that can connect to the spot monitor 102.

The sepsis risk factor data 704 includes measured vital signs data including heart rate, respiratory rate, blood pressure, or blood oxygen saturation data. In some further embodiments, the sepsis risk factor data 704 may also include data such as temperature and mean arterial pressure (MAP), and derived parameters such as shock index (SI) which is an assessment defined as heart rate divided by systolic blood pressure, and estimated cardiac output calculations. In some further embodiments, the sepsis risk factor data 704 may also include mobility data measured from an accelerometer patch or bed load cell sensors.

In some examples, sepsis risk assessment score 706 is based on a change over time, a standard deviation, a maximum value, or a difference from a 24 hour moving average in the data. In some examples, the sepsis risk assessment score 706 is based on a change in heart rate, respiratory rate, blood pressure, or blood oxygen saturation above or below a baseline level.

In some embodiments, the sepsis risk assessment score 706 is classified in a risk stratification as a low risk, a medium risk, or a high risk. In alternative embodiments, the sepsis risk assessment score 706 is calculated as a numerical value.

In some examples, in addition to calculating the sepsis risk assessment score 706, a classifier is generated to identify a trend in the sepsis risk assessment score 706 such as an increase or decrease in the sepsis risk assessment score 706. As an illustrative example, the classifier can be a symbol such as an upward arrow to indicate an increasing score and a downward arrow to indicate a decreasing score, or a color such as red to indicate an increasing score and green to indicate a decreasing score. Additionally, the sepsis risk assessment score 706 can be trended according to mean, minimum, maximum, difference between minimum and maximum, standard deviation, first calculation, last calculation, different between first and last calculation, difference between last calculation and calculation performed 1, 2, 4, 12, 24, or 72 hours ago, frequency of the calculations (i.e., was the calculation taken and how often?). Also, a linear regression trend, a quadratic regression trend, skewness (i.e., a measure of the asymmetry of the probability distribution of a real-valued random variable about its mean), and kurtosis (i.e., a measure of the tails of the probability distribution of a real-valued random variable) can be calculated for analyzing trends in the sepsis risk assessment score 706.

In some examples, the sepsis risk assessment score 706 is generated in a manner to prevent alarm fatigue. For example, the sepsis risk assessment score 706 can be compared to a threshold value specific to the patient (e.g., based upon the patient's prior history) or can be compared to a general threshold value associated with the patient's demographic (e.g., age, sex, etc.). When the sepsis risk assessment score exceeds the threshold value, or if the score is classified as a high risk, a notification is generated by the notification engine 306 to alert the caregivers that the patient is likely to develop sepsis. The notification drives an early intervention by the caregivers to mitigate the risks and costs associated with sepsis.

In contrast to Systemic inflammatory response syndrome (SIRS), Sequential Organ Failure Assessment (SOFA), and quick SOFA (qSOFA) scores, which are typically calculated every 1 to 4 hours using a limited number of factors, the sepsis risk assessment score 706 is continuously determined in near-real-time, such as every second, or every five seconds, or every minute, etc. Additionally, by automatically retrieving continuous data from the vital signs monitoring devices, electronic medical record system 108, and healthcare information systems 110, the sepsis risk assessment score 706 is calculated using additional factors not used to calculate the SIRS, SOFA, and qSOFA scores. Therefore, the sepsis risk assessment score 706 is also more refined and accurate over the SIRS, SOFA, and qSOFA scores which are based only on a limited number of factors. As an illustrative example, the sepsis risk assessment score 706 is calculated using risk factors including continuous vital signs and physiological data, laboratory data, clinical examination data, medications data, admissions data, and the like. The sepsis risk assessment score 706 may also be calculated using risk factors such as comorbidities, and recent medical procedures, operations, invasive medical devices, and other patient historical data. In some examples, the sepsis risk assessment score 706 is calculated using data from point of care devices such as, for example, a handheld blood analyzer that provides real-time measurements for lactate, glucose, procalcitonin, arterial blood gases, and similar parameters.

At least some of the risk factors which contribute to the sepsis risk assessment score 706 are summarized in Table 1. These risk factors are not exhaustive, and are provided as illustrative examples of the types of data that can be used to calculate the sepsis risk assessment score 706.

**Table 1**

| | **Risk Factor** |
|---|---|
| 1 | Heart rate |
| 2 | Heart Rate (Standard Deviation) |
| 3 | Heart rate (Max to date) |
| 4 | Heart rate (difference from 24-hr moving average) |
| 5 | Respiratory Rate |
| 6 | Respiratory Rate (Standard Deviation) |
| 7 | Respiratory Rate (Max to date) |
| 8 | Respiratory Rate (difference from 24-hr moving average) |
| 9 | Blood pressure |
| 10 | Blood pressure (Standard Deviation) |
| 11 | Blood pressure (Max to date) |
| 12 | Blood pressure (difference from 24-hr moving average) |
| 13 | SpO2 |
| 14 | SpO2 (Standard Deviation) |
| 15 | SpO2 (Max to date) |
| 16 | SpO2 (difference from 24-hr moving average) |
| 17 | Abdominal pain |
| 18 | Abdominal tenderness |
| 19 | Abscess |
| 20 | Acquired autoimmune disease |
| 21 | Active hyperemia |
| 22 | Acute lung injury |
| 23 | Age |
| 24 | Agitation |
| 25 | AIDS or HIV |
| 26 | Altered mental status |
| 27 | Anemia |
| 28 | Anxiety |
| 29 | Appendicitis |
| 30 | Arterial Ph |
| 31 | Aspiration |
| 32 | Asplenic |
| 33 | Autoimmune disease |
| 34 | Bacteremia |
| 35 | Bicarbonate |
| 36 | Bilirubin |
| 37 | Bone marrow transplant |
| 38 | Cancer |
| 39 | Capillary refill time |
| 40 | Cardiac output |
| 41 | Cellulitis |
| 42 | Chemotherapy |
| 43 | Cholangitis |
| 44 | Cholecystitis |
| 45 | Chronic infectious disease |
| 46 | Chronic Obstructive Pulmonary Disease |
| 47 | Cirrhosis |
| 48 | Colitis |
| 49 | Compromised cardiac output |
| 50 | Congestive heart failure |
| 51 | Corticosteriods |
| 52 | C-reactive protein |
| 53 | Creatinine |
| 54 | Cystitis |
| 55 | D-dimer |
| 56 | Decrease in daily functions |
| 57 | Decreased level of consciousness |
| 58 | Dehydration |
| 59 | Delirium |
| 60 | Dementia |
| 61 | Diabetes |
| 62 | Dialysis |
| 63 | Diaphoresis (sweating) |
| 64 | Diarrhea |
| 65 | Diverticulitis |
| 66 | Diverticulosis |
| 67 | Dyspnea |
| 68 | Encephalitis |
| 69 | Encephalopathy |
| 70 | Endocarditis |
| 71 | Fatigue |
| 72 | Fever |
| 73 | Gastroenteritis |
| 74 | Gastrointestinal bleed |
| 75 | Gastrointestinal tract infection |
| 76 | Glucose |
| 77 | Headache |
| 78 | Heart valve disorders |
| 79 | Hemaglobin |
| 80 | Hematacrit |
| 81 | Hyperlactatemia |
| 82 | Hypotension |
| 83 | Hypothermia |
| 84 | Ileus |
| 85 | Immunosuppressants |
| 86 | Increase in Creatinine |
| 87 | Increased pain |
| 88 | Inflammatory bowel disease |
| 89 | INR |
| 90 | IV drug abuse |
| 91 | Jaundice |
| 92 | Joint replacement |
| 93 | Lethargy |
| 94 | Leukopenia |
| 95 | Malaise |
| 96 | Mean arterial pressure |
| 97 | Meningitis |
| 98 | Mottling of skin |
| 99 | Nausea or vomiting (Emesis) |
| 100 | Neoplasm |
| 101 | Neutropenia |
| 102 | Neutrophils (Bands) |
| 103 | Nursing home resident |
| 104 | Oliguria |
| 105 | Organ transplant |
| 106 | Osteomyelitis |
| 107 | Ostomy |
| 108 | PaCO2 |
| 109 | PaO2 |
| 110 | PaO2/FiO2 |
| 111 | Pelvic Pain |
| 112 | Peripheral cyanosis |
| 113 | Petechial rash |
| 114 | Ph |
| 115 | Platelets |
| 116 | Pneumonia |
| 117 | Polydipsia |
| 118 | Polyuria |
| 119 | Poor tissue perfusion |
| 120 | Positive fluid balance |
| 121 | Postpartum |
| 122 | Pressure injury |
| 123 | Procalcitonin |
| 124 | Protein in urine |
| 125 | PTT |
| 126 | Pyelonephritis |
| 127 | Recent abortion |
| 128 | Recent c-section |
| 129 | Recent hospitalization |
| 130 | Recent past surgery |
| 131 | Recent Prior or Chronic Antibiotics |
| 132 | Recent vaginal delivery |
| 133 | Renal disease |
| 134 | Respiratory infection |
| 135 | Seizures |
| 136 | Septic arthritis |
| 137 | Sickle cell anemia |
| 138 | Soft tissue infection |
| 139 | Stupor |
| 140 | Surgery |
| 141 | Syncope |
| 142 | Tachycardia |
| 143 | Tachypnea |
| 144 | Transfer from ICU |
| 145 | Trauma |
| 146 | White blood cell count |
| 147 | Wound |

FIG. 8 illustrates example physical components of a computing device 1200, such as the computing device or devices used to implement aspects of the disclosure described above. As illustrated, the computing device 1200 includes at least one processor or central processing unit ("CPU") 1208, a system memory 1212, and a system bus 1210 that couples the system memory 1212 to the CPU 1208. The central processing unit 1208 is an example of a processing device.

The system memory 1212 includes a random access memory ("RAM") 1218 and a read-only memory ("ROM") 1220. A basic input/output system containing the basic routines that help to transfer information between elements within the computing device, such as during startup, is stored in the ROM 1220. The computing device further includes a mass storage device 1214. The mass storage device 1214 is able to store software instructions and data. The mass storage device 1214 is connected to the CPU 1208 through the system bus 1210.

The mass storage device 1214 and its associated computer-readable data storage media provide non-volatile, non-transitory storage for the computing device. Although the description of computer-readable data storage media contained herein refers to a mass storage device, such as a hard disk or CD-ROM drive, it should be appreciated by those skilled in the art that computer-readable data storage media can be any available non-transitory, physical device or article of manufacture from which the device can read data and/or instructions. The mass storage device 1214 is an example of a computer-readable storage device.

Computer-readable data storage media include volatile and non-volatile, removable and non-removable media implemented in any method or technology for storage of information such as computer-readable software instructions, data structures, program modules or other data. Example types of computer-readable data storage media include, but are not limited to, RAM, ROM, EPROM, EEPROM, flash memory or other solid-state memory technology, CD-ROMs, digital versatile discs ("DVDs"), other optical storage media, magnetic cassettes, magnetic tape, magnetic disk storage or other magnetic storage devices, or any other medium which can be used to store the desired information and which can be accessed by the computing device.

The computing device 1200 may operate in a networked environment using logical connections to remote network devices through a network 46, such as a local network, the Internet, or another type of network. The computing device 1200 connects to the network 46 through a network interface unit 1216 connected to the system bus 1210. The network interface unit 1216 may also connect to other types of networks and remote computing systems.

The computing device 1200 includes an input/output unit 1222 for receiving and processing input from a number of other devices, including a touch user interface display screen, or another type of input device. Similarly, the input/output unit 1222 may provide output to a touch user interface display screen, a printer, or other type of output device.

As mentioned above, the mass storage device 1214 and the RAM 1218 of the computing device 1200 can store software instructions and data. The software instructions include an operating system 1232 suitable for controlling the operation of the computing device 1200. The mass storage device 1214 and/or the RAM 1218 also store software instructions, that when executed by the CPU 1208, cause the computing device 1200 to provide the functionality discussed in this document, including the methods described herein.

Communication media may be embodied in the software instructions, data structures, program modules, or other data in a modulated data signal, such as a carrier wave or other transport mechanism, and includes any information delivery media. The term "modulated data signal" may describe a signal that has one or more characteristics set or changed in such a manner as to encode information in the signal. By way of example, communication media may include wired media such as a wired network or direct-wired connection, and wireless media such as acoustic, radio frequency (RF), infrared, and other wireless media.

The block diagrams depicted herein are just examples. There may be many variations to these diagrams described therein without departing from the spirit of the disclosure. For instance, components may be added, deleted or modified.

## Claims

1. A sepsis monitoring system comprising:
a processor and a non-transitory computer-readable storage medium storing instructions that, when executed by the processor, cause the sepsis monitoring system to:
determine an initial sepsis risk assessment score;
automatically and continuously update the sepsis risk assessment score using a sepsis risk assessment model that receives vital signs data, electronic medical records data, and admissions data to continuously update the sepsis risk assessment score;
determine whether the patient is likely to develop sepsis based on the updated sepsis risk assessment score; and
in response to determining that the patient is likely to develop sepsis, generate a notification to drive an early intervention by one or more caregivers.

2. The system of claim 1, wherein the vital signs data includes a change over time, a standard deviation, a maximum value, or a difference from a 24 hour moving average.

3. The system of either of claim 1 or claim 2, wherein the updated sepsis risk assessment score is based at least in part on heart rate, respiratory rate, blood pressure, or blood oxygen saturation.

4. The system of any preceding claim, wherein the updated sepsis risk assessment score is based at least in part on a change in heart rate, a change in respiratory rate, a change in blood pressure, and a change in blood oxygen saturation above or below a baseline level.

5. The system of any preceding claim, wherein the vital signs data is acquired from at least one of a spot monitor device, a wearable device, or a mattress pad device.

6. The system of any preceding claim, wherein the sepsis risk assessment score is classified in a risk stratification as low risk, medium risk, or high risk, and the notification is generated in response to the sepsis risk assessment score being classified as high risk or medium risk.

7. The system of any preceding claim, wherein the sepsis risk assessment score is a numerical value, and the notification driving the early intervention is generated in response to the sepsis risk assessment score exceeding a threshold value.

8. The system of any preceding claim, wherein the sepsis risk assessment model is built using one or more machine learning techniques.

9. The system of any preceding claim, wherein the notification is displayed on one or more workstations within a healthcare facility.

10. The system of any preceding claim, wherein the notification is displayed as an EMR plug-in.

11. A method of providing an early intervention for mitigating risks associated with sepsis, the method comprising activating a sepsis monitoring system to carry out the steps comprising:
determining an initial sepsis risk assessment score;
continuously receiving measured vital signs from a vital signs monitoring device;
automatically and continuously determining an updated sepsis risk assessment score based on at least the measured vital signs;
determining whether a patient is likely to develop sepsis based on the updated sepsis risk assessment score; and
in response to determining that the patient is likely to develop sepsis, generating a notification to drive an early intervention by one or more caregivers.

12. The method of claim 11, further comprising comparing the updated sepsis risk assessment score to a threshold value, and the generating the notification when the updated sepsis risk assessment score exceeds the threshold value.

13. The method of either claim 11 or claim 12 , further comprising using a sepsis risk assessment model to automatically and continuously determine the updated sepsis risk assessment score.

14. The method of any one of claim 11 to 13, further comprising continuously inputting vital signs data, electronic medical records data, and admissions data into the sepsis risk assessment model to continuously determine the sepsis risk assessment score.

15. The method of any one of claim 11 to 14, further comprising building the sepsis risk assessment model using one or more machine learning techniques.
